# EUROPEAN PATENT APPLICATION

(11) **EP 2 392 376 A1**
(43) Date of publication of application: **07.12.2011**
(21) Application number: 11166828.1
(22) Date of filing: 20.05.2011
(51) Int. Cl.: A61M 25/09

(54) **Guidewire**

(30) Priority: 03.06.2010 JP 2010127792
(71) Applicant: ASAHI INTECC CO., LTD., Nagoya-shi, Aichi 463-0024 (JP)
(72) Inventor: Tsunezumi, Atsushi, Nagoya-shi Aichi 463-0024 (JP)
(74) Representative: Winter, Brandl, Fürniss, Hübner, Röss, Kaiser, Polte - Partnerschaft

(57) **Abstract**

Guidewire (1) capable of measuring a lesion location present in a vascular channel, preventing perforation of a vessel or the like by improving flexibility of a lesion measuring part, measuring a lesion of a peripheral portion by improving insertability of the guidewire into the peripheral portion of a tubular organ such as a vessel, and accurately measuring a lesion location inside a vascular channel even under heartbeats. The guidewire includes a core shaft (2) and a coiled body (3) wound around a front end of the core shaft. The coiled body is made of a coil of strand. The coil of strand is formed by arranging at least one of a radiopaque coil of strand (31) and at least one of a radiolucent coil (32) of strand alternately in line and connecting them with each other.

## Description

### Cross Reference to Related Applications

This application is based on Japanese Patent Application No. 2010-127792 filed with the Japan Patent Office on June 3, 2010, the entire content of which is hereby incorporated by reference.

### Technical Field

The present invention relates to a guidewire.

### Background Art

Conventionally, various kinds of guidewires inserted in a tubular organ such as a vessel, a digestive tract, or a ureter or an intracorporeal tissue for use in measurement of a lesion location have been proposed.

For example, JP-T-7-500749 describes a guidewire with a measurement function in which a string of radiopaque markers is fixed at a front end of the guidewire by bonding by adhesive, welding, or soldering.
In a guidewire described in JP-T-10-513081, a radiolucent proximal end coil and a radiopaque distal end coil are provided at least at a tip portion of an elongated wire. A front end of the proximal end coil and a proximal end of the distal end coil abut on each other. The abutting portion is welded by YAG laser or excimer laser. This causes a distal end of the distal end coil and a proximal end of the proximal end coil to float with respect to the wire.

### Summary of Invention

However, in the guidewire described in JP-T-7-500749, the markers are fixed at a measuring part. Hence, since the front end of the guidewire is hardened, this front end may perforate a vessel or the like.
Also, in a case where the front end of the guidewire is hardened, followability of the guidewire to a peripheral portion of a tubular organ such as a vessel will be significantly lowered. This causes a problem of not being able to measure a lesion location at the peripheral portion of the tubular organ such as a vessel by the markers.
Further, in a case where the front end of the guidewire is hardened, the guidewire will move by heartbeats when a lesion location at a coronary artery or the like is to be measured. This also causes a problem of not being able to perform accurate measurement.

Also, in the guidewire described in JP-T-10-513081, the radiolucent proximal end coil and the radiopaque distal end coil are fixed by laser welding or the like. This drastically increases the stiffness of the coil part fixed by laser welding or the like. Accordingly, this guidewire cannot solve the problems caused by the technique described in JP-T-7-500749, either.

The present invention has been made to solve the foregoing problems, and an object of the present invention is to provide a guidewire capable of preventing perforation of a vessel or the like, measuring a peripheral portion of a tubular organ such as a vessel, and performing accurate measurement of a lesion location even under heartbeats.
This object is solved by a guidewire according to claim 1. Preferred embodiments thereof are subject-matter of dependent claims.

<1> An invention according to a first aspect is a guidewire including a core shaft and a coiled body wound around an outer circumference of the core shaft, wherein the coiled body is made of a coil of strand formed by arranging at least one of a radiopaque coil of strand and at least one of a radiolucent coil of strand alternately in line and connecting them with each other.

### Brief Description of Drawings

The foregoing and other objects, features, aspects and advantages of the invention will become more apparent from the following detailed description when taken in conjunction with the accompanying drawings.
Fig. 1 illustrates an overall view of a guidewire according to a first embodiment of the invention.
Fig. 2 illustrates an overall view of a guidewire according to a second embodiment of the invention.
Fig. 3 illustrates an overall view of a guidewire according to a third embodiment of the invention.
Fig. 4 illustrates an overall view of a guidewire according to a fourth embodiment of the invention.
Fig. 5 illustrates an overall view of a guidewire according to a fifth embodiment of the invention.
Fig. 6 illustrates an overall view of a guidewire according to a sixth embodiment of the invention.
Fig. 7 illustrates an overall view of a guidewire according to a seventh embodiment of the invention.
Fig. 8 illustrates an overall view of a guidewire according to an eighth embodiment of the invention.

### Description of Embodiments

Preferred embodiments of the present invention will be described below with reference to the accompanying drawings, in which like reference characters designate similar or identical parts throughout the several views thereof.
<1> An invention according to the first aspect is a guidewire including a core shaft and a coiled body wound around an outer circumference of the core shaft, wherein the coiled body is made of a coil of strand formed by arranging at least one of a radiopaque coil of strand and at least one of a radiolucent coil of strand alternately in line and connecting them with each other.

<2> An invention according to a second aspect is the guidewire according to the first aspect, wherein the respective radiolucent coils of strand have equal lengths.

<3> An invention according to a third aspect is the guidewire according to the second aspect, wherein the respective radiopaque coils of strand have equal lengths.

<4> An invention according to a fourth aspect is the guidewire according to any one of aspects 1 to 3, wherein the coiled body has a thin portion (i.e., a small diameter portion) and a thick portion (i.e., a large diameter portion) arranged alternately, the thin portion is formed of either one of the radiopaque and radiolucent coils of strand, and the thick portion is formed of the other one of the radiopaque and radiolucent coils of strand.

<5> An invention according to a fifth aspect is the guidewire according to the fourth aspect, wherein a coil of strand forming the thick portion has a higher flexural stiffness than a coil of strand forming the thin portion.

<6> An invention according to a sixth aspect is the guidewire according to the fourth or fifth aspect, wherein the thin portion is provided at a front end of the guidewire.

<1> As described above, in the guidewire according to the first aspect, the coiled body of the guidewire is made of a coil of strand formed by arranging the radiopaque coil(s) of strand and the radiolucent coil(s) of strand alternately in line and connecting them with each other. Thus, the flexibility of the coiled body is secured. This leads to improvement in insertability of the guidewire, and thus a lesion location in a thin vascular channel can be measured. The coil of strand making the body of the guidewire is formed by at least one of a radiopaque coil of strand and at least one of a radiolucent coil of strand which are arranged in line and connected with each other. Preferably, the coil of strand making the body of the guidewire is formed by a plurality of radiopaque coils of strand and a plurality of radiolucent coils of strand which are arranged alternately in line and connected with each other. In this case, the number of coils of strand of the one type (i.e., radiopaque or radiolucent) is identical to or unequal by one from the number of coils of strand of the other type (i.e., radiolucent or radiopaque).

<2> In the guidewire according to the second aspect, the respective radiolucent coils of strand have equal lengths. Hence, the distances between radiopaque portions can be uniform. Accordingly, a lesion location can be measured accurately.

<3> In the guidewire according to the third aspect, the respective radiopaque coils of strand have equal lengths. Hence, the lengths of the radiopaque portions can be uniform. Accordingly, a lesion location can be measured accurately.

<4> In the guidewire according to the fourth aspect, the coiled body has the (small diameter, or) thin portion and the (large diameter, or) thick portion arranged alternately. Also, the thin portion is formed of either one of the radiopaque and radiolucent coils of strand. Further, the thick portion is formed of the other one of the radiopaque and radiolucent coils of strand. In this manner, the coiled body of the guidewire is provided with the thin portion and the thick portion respectively corresponding to the radiopaque portion and the radiolucent portion. This can prevent movement of the guidewire due to heartbeats at the time of measurement of a lesion location in a vascular channel.

<5> In the guidewire according to the fifth aspect, the thick portion of the coiled body is formed of a coil of strand having a high flexural stiffness. Thus, the thick portion of the coiled body can be formed easily.
Also, by forming the thick portion of the coiled body of a coil of strand having a high flexural stiffness, an engaging force of the guidewire at a stenosis lesion location can be enhanced. This can prevent movement of the guidewire due to heartbeats at the time of measurement of a lesion location in a vascular channel.

<6> In the guidewire according to the sixth aspect, the front end of the coiled body is the thin portion. This leads to improvement in insertability of the guidewire. Accordingly, a lesion location in a thin vascular channel can be measured.

Hereinafter, preferred embodiments of a guidewire according to the present invention will be described with reference to the drawings.

### <First Embodiment>

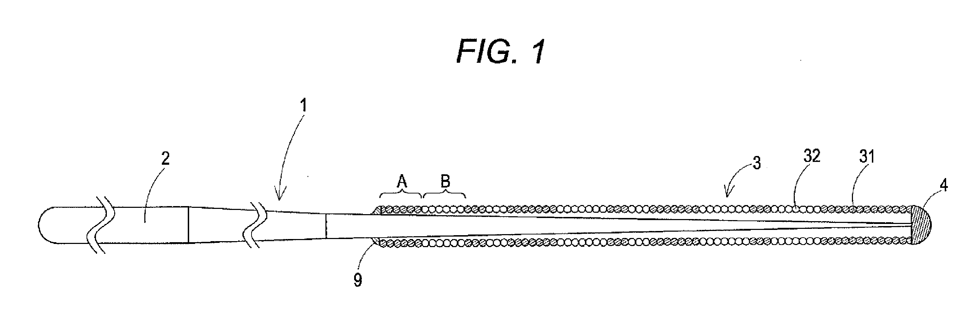

Fig. 1 illustrates an overall view of a guidewire according to a first embodiment of the invention.

It is noted that, in Fig. 1, the left side is referred to as "a proximal side," and the right side is referred to as "a front side" for convenience of explanation.
Also, in Fig. 1, a guidewire 1 is shortened in the longitudinal direction to show the entirety schematically for ease of understanding. Accordingly, the entire dimension differs from the actual one.

In Fig. 1, the guidewire 1 has a core shaft 2 and a coiled body 3 covering the tip portion of the core shaft 2. The tip portion of the core shaft 2 and the tip portion of the coiled body 3 are fixed at a most distal portion 4. The proximal portion of the coiled body 3 is fixed to the core shaft 2 by a brazed portion 9 at a position on the proximal side of the most distal portion 4.

A material for the core shaft 2 is not particularly limited but can be a material such as stainless steel (SUS 304), a super elastic alloy such as an Ni-Ti alloy, or a piano wire.

A material for the most distal portion 4 fixing the core shaft 2 and the coiled body 3 and for the brazed portion 9 are not particularly limited but can be an aluminum alloy brazing material, a silver brazing material, a gold brazing material, zinc, an Sn-Pb alloy, a Pb-Ag alloy, an Sn-Ag alloy, or the like.

The coiled body 3 is formed by winding a coil of strand. This coil of strand is formed by arranging radiopaque coils of strand 31 and radiolucent coils of strand 32 alternately in line and connecting them with each other. The coiled body 3 has radiopaque portions A formed of the radiopaque coils of strand 31 and radiolucent portions B formed of the radiolucent coils of strand 32.

In this manner, the coiled body 3 is formed of a coil of strand. This coil of strand is formed by arranging the radiopaque coils of strand 31 and the radiolucent coils of strand 32 alternately in line and connecting them with each other. By doing so, a highly flexible guidewire with a measurement function can be produced.
Also, since the guidewire is highly flexible, perforation of a vessel or the like can be prevented. Further, since followability of the guidewire to a peripheral portion of a tubular organ such as a vessel can be heightened, a lesion location at the peripheral portion can be measured.

A material for the radiopaque coils of strand 31 is not particularly limited but can be gold, platinum, tungsten, an alloy containing these elements (e.g., a platinum-nickel alloy), or the like.

A material for the radiolucent coils of strand 32 is not particularly limited but can be stainless steel (SUS 304, SUS 316, or the like), a super elastic alloy such as an Ni-Ti alloy, a piano wire, or the like.

The coiled body 3 can be produced by the following method, for example.
The radiopaque coils of strand 31 and the radiolucent coils of strand 32 are arranged alternately in line. Subsequently, the endfaces of the respective coils of strand are connected with each other by means of welding such as butt welding, brazing, bonding, or the like. By doing so, a coil of strand in which the radiopaque coils of strand 31 and the radiolucent coils of strand 32 are arranged alternately is formed.
Meanwhile, for connection between the radiopaque coils of strand 31 and the radiolucent coils of strand 32, a component that improves connectivity (brazing material or the like) or a connecting member such as a tubular member may be used.

Next, the coil of strand formed in such a manner is wound around a core rod and formed in a coiled shape. Thereafter, the core rod is pulled out. By doing so, the coiled body 3 having the radiopaque portions A and the radiolucent portions B can be obtained.

The coiled body 3 may be produced not only by this method but also by other methods (e.g., a coiling pin method).

Also, before being wound in a coiled shape, the coil of strand for the coiled body 3 can be thinned by die drawing. By thinning the coil of strand for the coiled body 3, the coiled body 3 can be more flexible.

Although not shown in Fig. 1, a lubricant for heightening lubricity of the coiled body 3 can be coated on the coiled body 3 of the guidewire 1. The lubricant is not particularly limited as long as it increases lubricity of the guidewire. Examples of the lubricant include hydrophobic lubricants such as silicone oil and fluorine resin, and hydrophilic lubricants such as polyvinyl pyrrolidone, polyvinyl alcohol, maleic anhydride copolymer, and hyaluronic acid.

By coating such a lubricant on the coiled body 3, lubricity of the guidewire 1 is increased. Accordingly, insertability of the guidewire 1 can be further improved. This enables easy measurement of a lesion location at a peripheral portion of a tubular organ such as a vessel.

### <Second Embodiment>

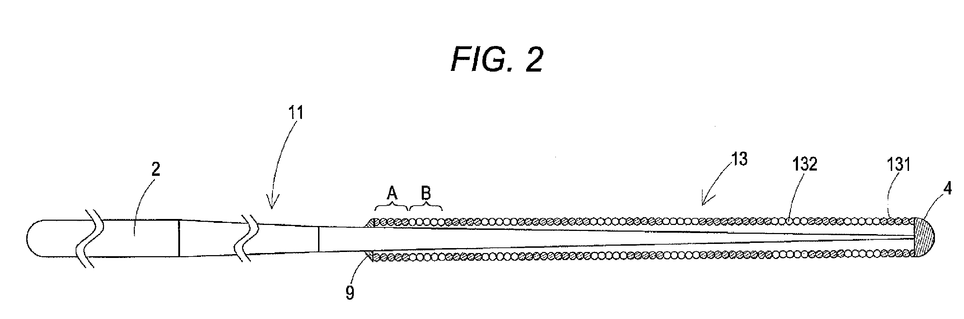

Next, a guidewire according to a second embodiment will be described with reference to Fig. 2 mainly on the difference from the first embodiment.
In Fig. 2 as well as in Fig. 1, a guidewire 11 is shortened in the longitudinal direction to show the entirety schematically for ease of understanding. Accordingly, the entire dimension differs from the actual one.

A coiled body 13 of the guidewire 11 is formed by arranging radiopaque coils of strand 131 and radiolucents coil of strand 132 alternately in line and connecting them with each other. The radiolucent coils of strand 132 are formed to have equal lengths to one another. Thus, radiolucent portions B have equal lengths to one another. Hence, the distances between radiopaque portions A are uniform. Accordingly, a lesion location can be measured accurately.

Meanwhile, the length of the radiopaque portions A can be changed in accordance with a lesion location for which the guidewire is to be used. The length of a therapeutic device such as a stent that dilates a stenosis part differs depending on a treated part to which the guidewire is to be applied (e.g., a coronary artery, a peripheral vessel in a lower extremity or the like, a renal artery, and a carotid artery). Accordingly, by designing the length of the radiopaque portions A in accordance with a lesion location to which the guidewire is to be applied, appropriate measurement of a lesion location can be performed.

### <Third Embodiment>

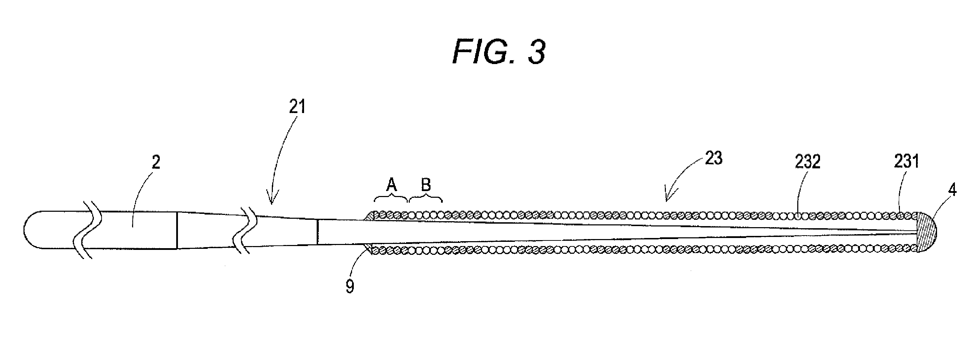

Next, a guidewire according to a third embodiment will be described with reference to Fig. 3 mainly on the difference from the first embodiment.
In Fig. 3 as well as in Fig. 1, a guidewire 21 is shortened in the longitudinal direction to show the entirety schematically for ease of understanding. Accordingly, the entire dimension differs from the actual one.

A coiled body 23 of the guidewire 21 is formed by arranging radiopaque coils of strand 231 and radiolucent coils of strand 232 alternately in line and connecting them with each other. As shown in Fig. 3, the radiopaque coils of strand 231 constituting radiopaque portions A and the radiolucent coils of strand 232 constituting radiolucent portions B have equal lengths. Thus, the distances between the radiopaque portions A (the lengths of the radiolucent portions B) are equal to one another, and the lengths of the radiopaque portions A are equal to one another. Accordingly, a lesion location can be measured more accurately.

In the configuration shown in Fig. 3, the radiopaque portions A and the radiolucent portions B have equal lengths to each other. However, the embodiment is not limited to this configuration, and the radiopaque portions A and the radiolucent portions B may have different lengths from each other (for example, the radiopaque portions A are made to be longer than the radiolucent portions B) in accordance with a lesion location to which the guidewire is to be applied for the reasons described in the second embodiment.

### <Fourth Embodiment>

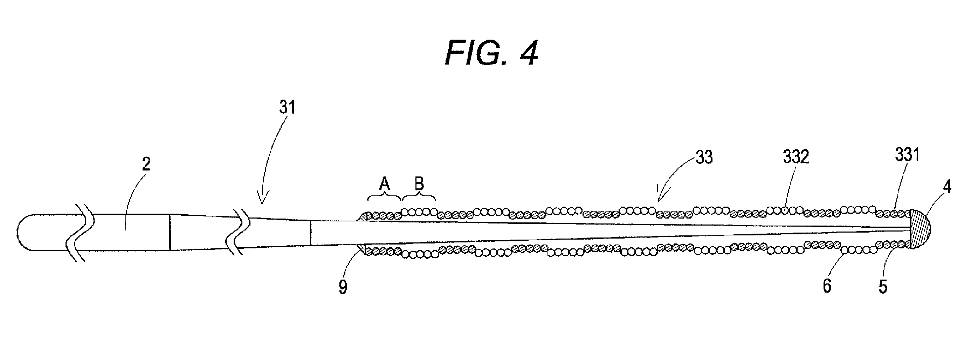

Next, a guidewire according to a fourth embodiment will be described with reference to Fig. 4 mainly on the difference from the first embodiment.
In Fig. 4 as well as in Fig. 1, a guidewire 31 is shortened in the longitudinal direction to show the entirety schematically for ease of understanding. Accordingly, the entire dimension differs from the actual one.

A coiled body 33 of the guidewire 31 is formed by arranging radiopaque coils of strand 331 and radiolucent coils of strand 332 alternately in line and connecting them with each other. The coiled body 33 has thin portions 5 and thick portions 6, which are arranged alternately.

In the configuration in Fig. 4, the thin portions 5 are made of the radiopaque coils of strand 331 and form radiopaque portions A while the thick portions 6 are made of the radiolucent coils of strand 332 and form radiolucent portions B.

Such a configuration contributes to increase in a locking force at a lesion location. This prevents the guidewire 31 from undesirably moving from the lesion location by heartbeats or the like, for example. Accordingly, the lesion location can be measured more reliably.

The coiled body 33 having the thin portions 5 and the thick portions 6 can be produced by the following method.
The length of the radiopaque coils of strand 331 and the length of the radiolucent coils of strand 332 are set in advance so that the radiopaque portions A and the radiolucent portions B may have equal lengths when they are wound in a coiled shape. A coil of strand is formed with use of the coils of strand 331 and 332 set in such a manner.

Next, thin portions and thick portions are formed on a core rod in advance in accordance with the lengths of the radiopaque portions A and the radiolucent portions B to be formed before the coil of strand is wound in a coiled shape. The coil of strand is wound around the core rod so that the radiopaque coils of strand may be wound around the thin portions of the core rod, for example. Thereafter, tensile stresses are given to both the ends of the core rod to thin the core rod. Thereafter, the core rod is pulled out. In this manner, the coiled body 33 having the thin portions 5 and the thick portions 6 respectively corresponding to the radiopaque portions A and the radiolucent portions B can be produced.

Also, as a producing method that further improves production efficiency, there is a method of setting the flexural stiffness of the radiopaque coils of strand 331 and the flexural stiffness of the radiolucent coils of strand 332 differently and winding a coil of strand made by connecting them with each other on a core rod having a uniform outer diameter.

That is, a part formed of the coil of strand with a high flexural stiffness naturally forms the thick portions 6 by springback. By doing so, the thin portions 5 and the thick portions 6 can be formed easily.
Also, by forming the thick portions of the coil of strand with the high flexural stiffness, these thick portions 6 are locked at a lesion location more easily. This can further prevent the guidewire 31 from undesirably moving by heartbeats or the like. Accordingly, the lesion location can be measured more accurately.

Combination of materials for the coil of strand (the radiopaque coils of strand 331 and the radiolucent coils of strand 332) at the time of conducting the method for forming the thin portions 5 and the thick portions 6 on the coiled body 33 by springback is not particularly limited as long as the stiffness of the coils of strand 331 differs from that of the coils of strand 332. For example, SUS 316 with a high flexural stiffness can be used as a material for the radiolucent coils of strand 332 while a platinum-nickel alloy with a low flexural stiffness can be used as a material for the radiopaque coils of strand 331.

Also, in the configuration shown in Fig. 4, the thin portion 5 is the radiopaque portion A while the thick portion 6 is the radiolucent portion B. However, the embodiment is not limited to this configuration, and the thin portion 5 may be the radiolucent portion B while the thick portion 6 may be the radiopaque portion A.

Also, in the configuration in which the thin portions 5 and the thick portions 6 are provided, it is preferable to provide a thin portion 5 at the front end of the guidewire 31 as shown in Fig. 4. Such a configuration leads to improvement in insertability of the guidewire 31 into a peripheral portion of a tubular organ such as a vessel, and thus a lesion location at the peripheral portion can be measured.

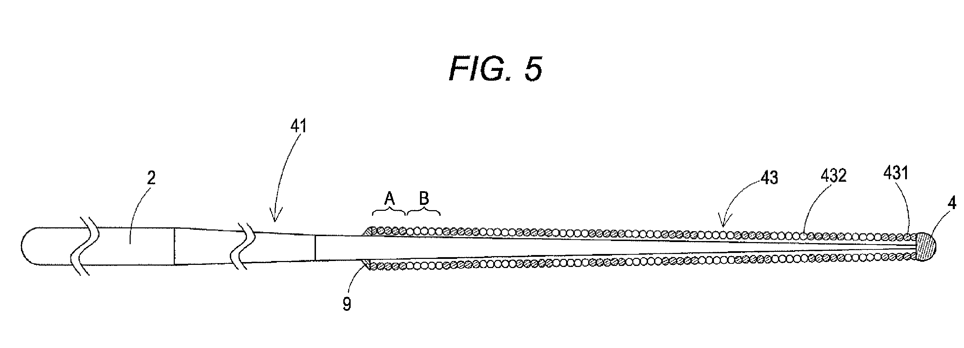

In an example of other embodiments, a configuration in which a coiled body 43 formed of radiopaque coils of strand 431 and radiolucent coils of strand 432 is tapered can be adopted as shown in Fig. 5. Since the coiled body 43 is tapered, the flexibility of the coiled body 43 is improved. This leads to improvement in insertability of a guidewire 41 into a peripheral portion of a tubular organ such as a vessel, and thus a lesion location at the peripheral portion can be measured.

With such a configuration, the outer diameters of radiopaque portions A gradually decrease toward the front end of the guidewire 41. Thus, a reference point for measurement is clearer under radiographic guidance than in a configuration in which the radiopaque portions A have equal diameters over the entire length of the coiled body. Accordingly, a lesion location can be measured accurately.

Although not shown in the figures, thin portions 5 and thick portions 6 similar to those shown in Fig. 4 may be provided on the coiled body 43 shown in Fig. 5. With such a configuration, a locking force at a lesion location of a peripheral portion can be given to the guidewire 41.

Meanwhile, the tapered shape shown in Fig. 5 may be formed over the entire length of the coiled body 43 or at a part of the coiled body 43.

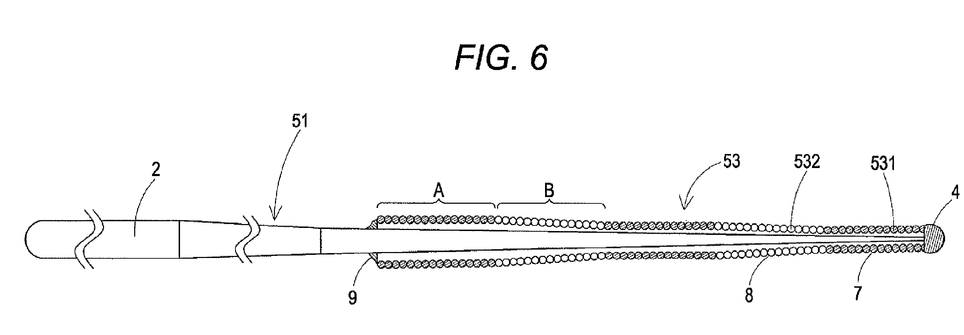

Also, as another example of a tapered shape of the coiled body, radiopaque portions A of a coiled body 53 (made of radiopaque coils of strand 531) may be equal diameter portions 7 while radiolucent portions B (made of radiolucent coils of strand 532) may be tapered portions 8, in which the coil outer diameter decreases toward the front end of a guidewire 51, as shown in Fig. 6. In this configuration, the coil outer diameter decreases in a stepwise manner toward the front end of the guidewire 51.

Since this leads to improvement in the flexibility of the coiled body 53, insertability of the guidewire 51 into a peripheral portion of a tubular organ such as a vessel is improved. In addition, the outer diameters of the radiopaque portions A decrease in a stepwise manner toward the front end of the guidewire. Thus, a reference point for measurement is clearer under radiographic guidance than in a configuration in which the radiopaque portions A have equal diameters over the entire length of the coiled body. Accordingly, a lesion location can be measured accurately.

Meanwhile, the radiopaque portions A may be tapered. Also, the radiolucent portions B may be equal diameter portions. Further, a part of the radiopaque portions A or the radiolucent portions B may be tapered or an equal diameter portion.

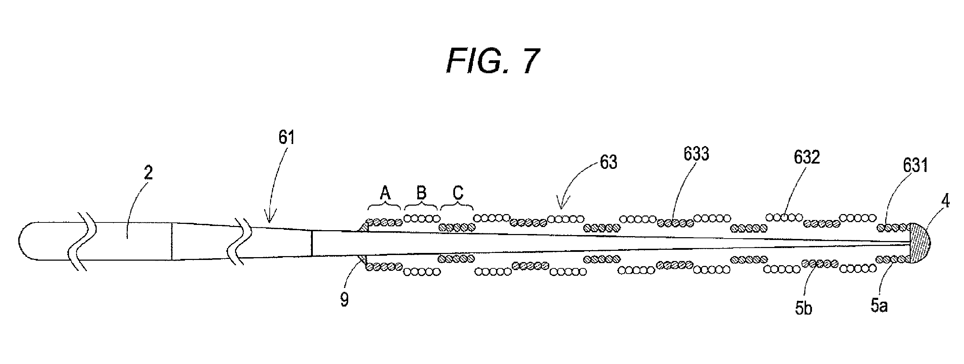

In another embodiment, thin radiopaque portions may be configured to further have two coil outer diameters (small thin portions 5a and large thin portions 5b) as shown in Fig. 7.

The small thin portions 5a are made of radiopaque coils of strand 631 and form radiopaque portions C. Also, the large thin portions 5b are made of radiopaque coils of strand 633 and form radiopaque portions A.

Such a configuration contributes to improvement in a locking force at a lesion location. Also, due to existence of the radiopaque portions C and the radiopaque portions A having different outer diameters, the marker thickness can be changed under radiographic guidance. Thus, since a reference point for measurement is clearer, a lesion location can be measured more accurately.

Meanwhile, the radiopaque portions A and the radiopaque portions C may be formed over the entire length of a coiled body 63 as shown in Fig. 7 or at a part of the coiled body 63 in the longitudinal direction.

Also, it is preferable to locate a small thin portion 5a at the front end of a guidewire 61. Such a configuration leads to improvement in insertability of the guidewire 61 into a peripheral vessel or the like.
Also, the radiopaque coils of strand 631 and 633 may be made of different materials from each other.

Also, although not shown in the figure, a radiolucent portion B made of a radiolucent coil of strand 632 may be provided with a portion having a different outer diameter. Such a configuration contributes to further increase in a locking force at a lesion location. This prevents the guidewire 61 from undesirably moving from the lesion location by heartbeats or the like, for example. Accordingly, the lesion location can be measured more reliably.

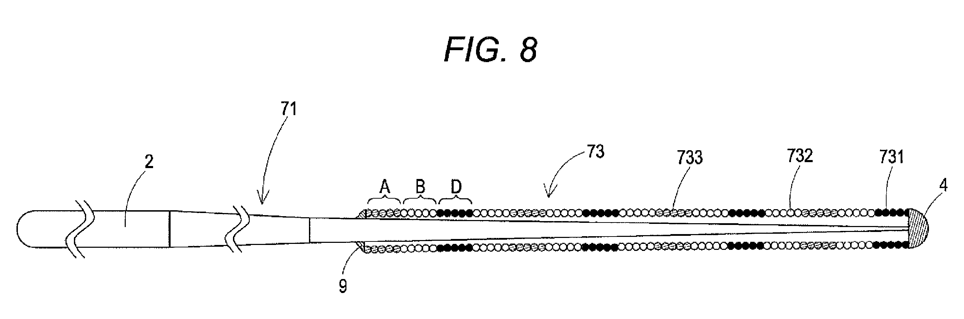

In another embodiment, a coiled body 73 of a guidewire 71 may be constituted by radiolucent portions B, highly radiopaque portions D, and radiopaque portions A, as shown in Fig. 8. The radiolucent portions B are made of radiolucent coils of strand 732. The highly radiopaque portions D are made of coils of strand 731 with a high radiopaque characteristic. The radiopaque portions A are made of coils of strand 733. The coils of strand 733 have a radiopaque characteristic and are less radiopaque than the radiopaque coils of strand 731.

By setting the radiopaque characteristics of the radiopaque portions in a coil of strand differently in this manner, the coiled body having plural kinds of radiopaque portions with different brightness levels can be formed. Thus, markers show difference in brightness under radiographic guidance. This makes a reference point for measurement clearer. Accordingly, by using the guidewire 71 having such a coiled body 73, a lesion location can be measured more accurately.

Examples of a method of setting the radiopaque characteristics of the radiopaque portions in the coil of strand differently include a method of forming the coil of strand by metals with different radiopaque characteristics and a method of increasing or decreasing the amount of an element with a radiopaque characteristic contained in an alloy forming the coil of strand.

Meanwhile, the brightness differences in the radiopaque portions may be provided over the entire length of the coiled body 73 as shown in Fig. 8 or at a part of the coiled body 73 in the longitudinal direction.

Also, the configuration shown in Fig. 7 having the radiopaque portions A (large thin portions 5b) and the radiopaque portions C (small thin portions 5a) with different diameters may also be provided with the aforementioned brightness difference. That is, the radiopaque characteristics of the large thin portions 5b and the small thin portions 5a may be set differently.

Also, although not shown in the figures, an intermediate brazed portion may be provided between the core shaft and the coiled body, but not to the extent of impairing the flexibility of the coiled body. By providing such an intermediate brazed portion, the coiled body can be prevented from separating from the core shaft. This can heighten safety of the guidewire.
Meanwhile, a material for the intermediate brazed portion may be the same as one for the most distal portion 4 and the brazed portion 9.
In the aforementioned embodiments, a coil of strand forming a coiled body is formed by arranging at least one of a radiopaque coil of strand and at least one of a radiolucent coil of strand alternately in line and connecting them with each other. In this regard, the number of the radiopaque coils of strand and the number of the radiolucent coils of strand may be identical or may be different from each other. Also, either one of the two kinds of coils of strand may be plural, and the other one may be single.
While the invention has been shown and described in detail, the foregoing description is in all aspects illustrative and not restrictive. It is therefore understood that numerous modifications and variations can be devised without departing from the spirit and scope of the invention.

### Reference Signs List

- 1, 11, 21, 31, 41, 51, 61, 71: guidewire
- 2: core shaft
- 3, 13, 23, 33, 43, 53, 63, 73: coiled body
- 4: most distal portion
- 5: thin portion
- 6: thick portion
- 7: equal diameter portion
- 8: tapered portion
- 9: brazed portion
- A: radiopaque portion
- B: radiolucent portion

## Claims

1. A guidewire (1; 11; 21; 31; 41; 51; 61; 71) comprising:
a core shaft (2); and
a coiled body (3; 13; 23; 33; 43; 53; 63; 73) wound around an outer circumference of the core shaft (2), **characterized in that**
the coiled body (3; 13; 23; 33; 43; 53; 63; 73) is made of a coil of strand formed by arranging at least one of a radiopaque coil of strand (31; 131; 231; 331; 431; 531; 631, 633; 731, 733) and at least one of a radiolucent coil of strand (32; 132; 232; 332; 432; 532; 632; 732) alternately in line and connecting them with each other.

2. The guidewire (11; 21; 31) according to claim 1, wherein the respective radiolucent coils of strand (132; 232; 332) have equal lengths.

3. The guidewire (21; 31) according to claim 1 or 2, wherein the respective radiopaque coils of strand (231; 331) have equal lengths.

4. The guidewire (31) according to any one of claims 1 to 3, wherein the coiled body (33) has at least one of a thin portion (5) and at least one of a thick portion (6) arranged alternately,
the at least one thin portion (5) is formed of either one of the radiopaque and radiolucent coils of strand (331, 332), and
the at least one thick portion (6) is formed of the other one of the radiopaque and radiolucent coils of strand (331, 332).

5. The guidewire (31) according to claim 4, wherein a coil of strand forming the thick portion (6) has a higher flexural stiffness than a coil of strand forming the thin portion (5).

6. The guidewire (31) according to claim 4 or 5, wherein a thin portion (5) is provided at a front end of the guidewire (31).
